# EUROPEAN PATENT APPLICATION

(11) **EP 4 775 692 A1**
(43) Date of publication of application: **15.07.2026**
(21) Application number: 23951240.3
(22) Date of filing: 14.09.2023
(51) Int. Cl.: C12Q 1/6895

(54) **SNP MARKER AND IDENTIFICATION METHOD FOR IDENTIFYING FEMALE AND MALE SIRAITIAE FRUCTUS PLANTS**

(30) Priority: 05.09.2023 CN 202311140049
(71) Applicant: GUILIN GFS MONK FRUIT CORPORATION, Guilin, Guangxi 541006 (CN)
(72) Inventor: WU, Wei, Guilin, Guangxi 541006 (CN); SUN, Shuku, Guilin, Guangxi 541006 (CN); ZHANG, Yanling, Guilin, Guangxi 541006 (CN); ZHANG, Xindan, Guilin, Guangxi 541006 (CN); LIU, Donglong, Guilin, Guangxi 541006 (CN); GUO, Lixia, Guilin, Guangxi 541006 (CN); LI, Mei, Guilin, Guangxi 541006 (CN); LAN, Fusheng, Guilin, Guangxi 541006 (CN); LAN, Feisi, Guilin, Guangxi 541006 (CN)
(74) Representative: CMS Cameron McKenna Nabarro Olswang LLP
(86) International application number: PCT/CN2023/118790
(87) International publication number: WO 2025/050423

(57) **Abstract**

By means of GWAS data association analysis of multiple years and multiple repetitions of phenotypic data of 125 varieties collected by Monk Fruit Corp. and wild natural varieties collected in the Guilin region, it is found that the sex-determining region is located in the 0-1 mbps range of chromosome 3 of the genome. Therefore, provided is an SNP region for early identification of female and male Siraitiae fructus plants. In this region, the female plant and the male plant have their own haplotypes. Finally, a group of haplotypes of male and female plants are screened out, and any SNP marker in the haplotypes can be used for development of SNP molecular markers and identification of male and female plants.

## Description

### TECHNICAL FIELD

The application relates to the field of genetic engineering and molecular biology, and the identification and application of a region involving sex determination for the development of dioeciousness in *Siraitia grosvenorii.*

### BACKGROUND

*Siraitia grosvenorii* is a dioecious plant, and its female and male plants cannot be identified with naked eyes in morphology, and can only be identified when flowering. Although there are relatively few seedlings used in production, mostly tissue culture propagation seedlings, the cultivation of seedlings is particularly important in the breeding of new high-yield and high sweet glycoside varieties. In order to improve the quality of seedlings, avoid the impact of viral diseases, and improve the root system and weak growth potential, if the gender of the plants cannot be distinguished during the seedling stage, then planting a large number of unscreened female and male plants will waste a significant amount of manpower and land resources. Therefore, the development of gender identification markers for seedings before rapid transplanting has important production significance, especially in enhancing the efficiency of hybrid superiority improvement in the breeding of *Siraitia grosvenorii.*

The previous method of selecting female and male plants usually involves selectively removing male plants during flowering to reduce the need for plant management. In 2013, Zhang Huixin et al. published a patent "SCAR molecular marker for identifying the gender of Siraitia grosvenorii*",* this marker has enabled the use of PCR to screen female and male plants in the early seedling stage. However, the amplification fragment in this patent cannot be determined to be related to sex-linked genes or genomic sex control loci. In addition, this marker may not be universally applicable, because this marker has not been verified in other *Siraitia grosvenorii* varieties in patent publication. In addition, the flux of this method is relatively slow and low.

The male-to-female ratio of *Siraitia grosvenorii* in the offspring is 1.3:1, and there are more male plants than female ones, which limits the promotion of hybrid seedlings in terms of economic value of planting. Due to the lack of a complete reference genome for *Siraitia grosvenorii,* the genetic polymorphism of *Siraitia grosvenorii* cannot be systematically compared and analyzed, making it impossible to screen and validate molecular markers. At the same time, the differences between female and male plants at the genomic level are also difficult to compare due to the lack of a reference genome, making it even more challenging to identify the decisive genes determining sex. Although the genetic ratio of male and female *Siraitia grosvenorii* is known, there is no similar model to explain the genetic mechanism of male and female *Siraitia grosvenorii.* In addition, when mapping the sex-determining genes of *Siraitia grosvenorii,* a complete reference genome is also required to enable the localization and identification of genes linked to the traits.

In 2018, Xia et al. assembled a *Siraitia grosvenorii* genome, but the genome was not assembled to the chromosome level, and there are more than 2000 contig fragments that could not be assembled were retained, making any gene mapping extremely difficult.

### SUMMARY

Due to lack of complete genome and sex identification markers, the improvement and large-scale screening of conventional breeding of dioecious *Siraitia grosvenorii* have been limited for a long time. In order to solve the existing problems in prior art, this application conducts whole genome splicing and assembly of a commercial variety from Monk Fruit Corp., compares the female and male plants of multiple different varieties on different chromosomes, with the aim of providing a haplotype distinguishing between female and male in a chromosome region, as well as SNP molecular markers and their applications in this haplotype.

The application can solve the problems of accuracy and reliability of sex identification in *Siraitia grosvenorii.* This technology can determine the gender of *Siraitia grosvenorii* plants at the seedling stage, avoiding the problem of wasting germplasm resources and costs. At the same time, this technology can be applied to different varieties of *Siraitia grosvenorii* plants, improving the universality and practicality of the study.

Specifically, the present application adopts the following technology,
1. A SNP marker used for identifying female and male *Siraitia grosvenorii* plants, wherein, the SNP marker is a locus with a p-value greater than 25.
2. The SNP marker according to item 1, wherein the nucleotide sequence of the SNP marker is as shown in SEQ ID NO: 1, located at position 122 of the nucleotide sequence, and the nucleotide represented by S in SEQ ID NO: 1 is G or C.
3. The SNP marker according to item 1 or 2, wherein, when the alleles at the diploid S position of the *Siraitia grosvenorii* are G and C, the *Siraitia grosvenorii* is a male plant, and when the alleles at the diploid S position of the *Siraitia grosvenorii* are C and C, the *Siraitia grosvenorii* is a female plant.
4. Primers for detecting the SNP marker according to any one of items 1-3, comprising,
   a forward primer 1:
      5'-GAAGGTGACCAAGTTCATGCTTCAACCATTGGGGATGGAG-3' ;
   a forward Primer 2:
      5'-GAAGGTCGGAGTCAACGGATTTCAACCATTGGGGATGGAC-3' ; and
      a reverse primer: 5'-GTAGAAGCGGCTGCTTTTACTGG-3'.
5. A detection reagent or kit for detecting a SNP marker according to any one of items 1-3, comprising the primers according to item 4.
6. Use of the SNP marker of any one of items 1 to 3, the primers of item 4, or the detection reagent or kit of item 5 in identifying or screening female and male plants of *Siraitia grosvenorii.*
7. Use of the SNP marker of any one of items 1 to 3, the primers of item 4 or the detection reagent or kit of item 5 in the marker assisted breeding of *Siraitia grosvenorii.*
8. Use of the SNP marker of any one of items 1 to 3, the primers of item 4 or the detection reagent or kit of item 5 in the hybrid breeding of *Siraitia grosvenorii.*
9. A method for distinguishing between female and male plants of *Siraitia grosvenorii,* wherein the SNP marker used for distinguishing between female and male plants of *Siraitia grosvenorii* in a test sample of *Siraitia grosvenorii* is detected to predict whether the *Siraitia grosvenorii* is female or male plant, wherein the SNP marker is the SNP marker of any one of items 1 to 3.
10. The method according to item 9, comprising the following steps,
   extracting the genomic DNA of *Siraitia grosvenorii* to be tested;
   using the genomic DNA of the *Siraitia grosvenorii* to be tested as a template, perform PCR amplification with the primers of item 4 to obtain the PCR amplification product;
   detecting the PCR amplification product, if the alleles at the diploid S position of the amplification product sequence are G and C, the *Siraitia grosvenorii* is a male plant; when the alleles at the diploid S position of the *Siraitia grosvenorii* are C and C, the *Siraitia grosvenorii* is a female plant.

### Effect of the Invention

This application uses 125 varieties collected by Monk Fruit Corp. and wild natural varieties collected in Guilin area to conduct GWAS data association analysis through years and multiple repeated phenotype data. The analysis reveals that the region determining sex is located in the 0-1 mbps interval on the 3rd chromosome of the genome. Therefore, this application provides an SNP region applicable for early identification of female and male plants of *Siraitia grosvenorii,* within which female and male plants have their own haplotypes. Finally, a group of haplotypes for female and male plants is screened out, and any SNP marker within it can be used for SNP molecular marker development and identification of female and male plants.

This application identified the respective common haplotypes of female and male plants through direct sequencing of 125 DNA samples of different varieties. The haplotypes of females and males are not limited by season, environment, or whether they are expressed or not.

The technical effect of the screening method in this application is efficient, fast and accurate, allowing for the determination of gender with 96% detection accuracy without the need to cultivate plants to flowering stage, which greatly reduces planting cost and increases the efficiency of hybrid breeding in *Siraitia grosvenorii.*

The research and application of this application alleviate the technical issues in the molecular breeding research of *Siraitia grosvenorii,* which include the lack of an accurate and complete reference genome, as well as effective and efficient molecular biological markers. The application of this technology will help improve the efficiency and quality of *Siraitia grosvenorii* breeding and promote the development of the *Siraitia grosvenorii* industry.

### BRIEF DESCRIPTION OF THE DRAWINGS

The drawings are intended for a better understanding of the present application and do not constitute an undue limitation to the present application. Among them:
Fig. 1 shows a Manhattan plot of SNP marker loci, with the x-axis representing chromosome numbers on the y-axis representing the -log P values from association analysis.
Fig. 2 shows the quantile plot of SNP loci for sex-trait association analysis.

### DETAIL DESCRIPTION

The following provides an explanation of the exemplary embodiments of the present application, including various details of these embodiments to aid in understanding, and they should be considered to be merely exemplary. Therefore, those skilled in the art should recognize that various changes and modifications can be made to the embodiments described herein without departing from the scope and spirit of the present application. Similarly, for clarity and conciseness, descriptions of well-known functions and structures are omitted in the following description.

The application provides SNP markers for identifying female and male plants of *Siraitia grosvenorii,* wherein the SNP markers are loci with p-value values greater than 25.

The term 'single nucleotide polymorphism' or 'SNP' refers to a DNA sequence change or genetic variant that occurs when a single nucleotide (e.g. adenine (A), thymine (T), cytosine (C), or guanine (G)) in the genome sequence is changed to another nucleotide.

The term 'p-value' is a parameter used to determine the result of a hypothesis test, and can also be compared using the rejection domain of different distributions. It was first proposed by R.A. Fisher.

The p-value is the probability that a more extreme result than the observed sample result will occur when the null hypothesis is true. If the P-value is small, it indicates that the probability of the occurrence of the null hypothesis is very low. However, if it does occur, according to the principle of small probability, there is a reason to reject the null hypothesis. The smaller the P-value, the more sufficient the reason to reject the null hypothesis. In summary, the smaller the P-value, the more significant the result.

In this application, p-value refers to a statistical significance indicator used in GWAS analysis to measure the association between genes or genetic variations and traits. A smaller P-value indicates that the observed association results is unlikely to be due to random factors, increasing the confidence in rejecting the null hypothesis. In this application, the p-value is expressed as the converted (-log₁₀(p)).

In this application, each SNP marker site with a p-value greater than 25 is considered to be an SNP associated with the identification of female and male plants of *Siraitia grosvenorii,* and can be used to distinguish between female and male plants of *Siraitia grosvenorii.*

*Siraitia grosvenorii* is the fruit of perennial vine plants of the cucurbitaceae family. It is also known as *Grosvener Siraitia,* Pseudobalsam pear, *Momordica grosvenorii,* Jinbuhuan, Luohanbiao, and Fructus Momordicae. It is known as the "fairy fruit". Its leaves are heart-shaped, and it is dioecious, flowering in summer, and bearing fruit in autumn. It is one of the first batch of medicinal and edible dual-use materials approved by the state, and its main function is to relieve cough and expel phlegm. The fruit has a high nutritional value and is rich in vitamin C (400-500 milligrams per 100 grams of fresh fruit) as well as glycosides, fructose, glucose, protein, lipids, etc. There are many varieties of *Siraitia grosvenorii,* with different names according to different classification methods. The SNP loci involved in this application can identify any variety of *Siraitia grosvenorii* without limitation, such as common varieties like Changtan fruit, green peel fruit, red hair fruit, tea mountain fruit, Lajiang fruit, winter melon fruit, etc.

In a specific embodiment, the nucleotide sequence of the SNP marker is shown as SEQ ID NO: 1, located at position 122 of the nucleotide sequence, wherein the nucleotide represented by S in SEQ ID NO: 1 is G or C.

ATTGTCGTCCTACCTATCTACAAAACAGCAGAGCCTTCAAAGTAGAAGCGGCTGCTT
TTACTGGATTATTAGAGAGAGAAGAAGTTGAGAAAATGAATGAACTGAGATTTGCTTGCA
TGCTSTCCATCCCCAATGGTTGATTTAATTCGTTTGGTAGGCAGAGATTGTAAAACATATAA
ACGAAATCTTTTGGGACATGATAAAAAATTAAATTTTCAGTTCCATGCTTTGGGTTGGGCC
CGTCATATTCTTATTCAACACACGTACGGGGCGCCT

In a specific embodiment, when the alleles at the diploid S position of the *Siraitia grosvenorii* are G and C, the *Siraitia grosvenorii* is a male plant, and when the alleles at the diploid S position of the *Siraitia grosvenorii* diploid are C and C, the *Siraitia grosvenorii* is a female plant.

The application provides a set of primers for identifying SNPs of female and male *Siraitia grosvenorii* plants, including forward primers and reverse primer, and the primer set includes primer pairs for detecting the SNP marker loci mentioned above.

In a specific embodiment, exemplary primer pairs are shown below, which include:
forward primer 1, as shown in SEQ ID NO: 2,
   5'-GAAGGTGACCAAGTTCATGCTTCAACCATTGGGGATGGAG-3' ;
forward primer 2, as shown in SEQ ID NO: 3,
   5'-GAAGGTCGGAGTCAACGGATTTCAACCATTGGGGATGGAC-3';
reverse primer, as shown in SEQ ID NO: 4,
   5'-GTAGAAGCGGCTGCTTTTACTGG-3'.

For the specific base sequences of the exemplary primer pairs mentioned above, as long as they can specifically recognize their respective specific recognition regions under PCR implementation conditions (preferably without annealing or self-annealing between primers used in a single reaction vessel), one or more bases can be replaced with other bases, or one or more bases can be added at the 3 'or 5' end. Here, the term "more" refers to, for example, 2-3. When adding one or more bases to a primer, it is preferred to add them to the 5 'end of the primer.

The identity of the base sequence obtained by substituting one or more bases in the specific base sequences of the primer exemplified above with other bases, to the base sequence before substitution (i.e., the base sequence indicated by the sequence number) is preferably 70% or more, more preferably 75% or more, more preferably 80% or more, more preferably 85% or more, more preferably 90% or more, and even more preferably 95% or more.

According to the examples of the application, the above primers have very good specificity, and they enable accurate and efficient PCR amplification of the fragment containing the SNP marker loci related to the tested *Siraitia grosvenorii.*

This application provides a detection reagent or kit for the SNP markers as described in any one of the above, comprising any primer as described above.

In a specific embodiment, the kit further comprises reagents for extracting genomic DNA from samples and reagents for PCR reaction using the primers.

In a specific embodiment, the reagent for extracting genomic DNA from samples can use existing kits.

In one embodiment, the reagents for PCR reaction using the primer set can be selected from the group consisting of: DNA polymerase, PCR buffer, dNTP mixture, and aqueous medium, or from pre-mixed solutions of the aforementioned reagents. For example, the pre-mixed solution is selected from EXTaq(HS): Premix Ex TaqTMHot Start Version, takara (RR030Q).

The PCR buffer typically provides the most suitable enzymatic reaction conditions for the PCR system. The buffer solution only needs to play the above role.

The dNTP mixture is usually used as a raw material in DNA synthesis, which can specifically include dATP, dGTP, dTTP, dCTP, etc.

The aqueous medium can usually be used to adjust the concentration of each component in the PCR system, and can usually be used as a dilution solvent.

The application further provides the use of SNP markers, primers, or detection reagents or kits as described in any one of the above in identifying or screening female and male plants of *Siraitia grosvenorii.*

The application further provides the use of SNP markers, primers, or detection reagents or kits as described in any of the above in the marker assisted breeding of *Siraitia grosvenorii.*

The application further provides the use of SNP markers, primers or detection reagents or kits as described in any of the above in the hybrid breeding of *Siraitia grosvenorii.*

The detection method for the SNP markers in this application is not specifically limited. SNP detection can be achieved through techniques such as sequencing, single-strand conformation polymorphism polymerase chain reaction (PCR-SCP), restriction fragment length polymorphism polymerase chain reaction (PCR-RFLP), and time-of-flight mass spectrometry. Among them, sequencing is a detection technology with high accuracy, strong flexibility, high throughput, and short detection cycle. It only requires designing a pair of primers flanking the SNP locus, amplifying a product of 400-700bp, and then directly detecting the genotype of the SNP locus through sequencing.

The application further provides a method for distinguishing between female and male plants of *Siraitia grosvenorii,* wherein, the SNP marker used for distinguishing between female and male plants of *Siraitia grosvenorii* in a test sample of *Siraitia grosvenorii* to predict whether the *Siraitia grosvenorii* is female or male plant, wherein the SNP marker is the SNP marker as described in any one of the above mentioned items.

In a specific embodiment, the identification method comprises the following steps:
extracting the genomic DNA of *Siraitia grosvenorii* to be tested;
using the genomic DNA of the *Siraitia grosvenorii* to be tested as a template, perform PCR amplification with the primers to obtain the PCR amplification product;
detecting the PCR amplification product, if the alleles at the diploid S position of the amplification product sequence are G and C, the *Siraitia grosvenorii* is a male plant; when the alleles at the diploid S position of the *Siraitia grosvenorii* diploid are C and C, the *Siraitia grosvenorii* is a female plant.

### EXAMPLE

### Example 1. Acquisition of SNP markers

### 1. Sample collection and processing

Monk Fruit Corp. has preserved 125 different varieties over the past 20 years, including commercial varieties and wild varieties collected in Guilin area. In this application, these *siraitia grosvenorii* germplasm resources were sequenced, 102 of which were female plants and 17 were male plants. They were cultivated, and the born tender buds were selected for DNA extraction.

### 2. The DNA extraction and purification methods used the most commonly used CTAB method in plants.

### 3. Screening and identification of SNP molecular markers:

### a. High throughput sequencing

The 125 *Siraitia grosvenorii* varieties mentioned above were deeply sequenced by high-throughput second-generation resequencing. The average sequencing depth of each locus reached 60×, and six million high MAF polymorphism loci were obtained.

At the same time, a *Siraitia grosvenorii* cultivar MFC0009 of a male lineage underwent third-generation sequencing, including HIFI and Hi-C sequencing, for chromosome-level assembly.

### b. Genome assembly

After third-generation sequencing and assembly, the genome of *Siraitia grosvenorii* was successfully assembled into a complete form and achieved chromosome-level assembly. The entire genome was 321 Mbps long.

In this field, whole genome sequencing and chromosome assembly of *Siraitia grosvenorii* have not been achieved until now. Based on more than 100 germplasm resources of *Siraitia grosvenorii* preserved by the applicant over the past 20 years, extensive sequencing and assembly efforts have culminated in the complete assembly of the *Siraitia grosvenorii* genome, and successfully assembling it to the chromosome level, which was the first time in this field. The total length of the whole genome was 321 Mbps, and the spliced genome and chromosomes were also determined by the applicant for the first time.

Although *Siraitia grosvenorii* has been cultivated for more than a century, its complete genome sequence has not been completely assembled in the past 20 years. In 2018, Mian Xia et.al. released an improved *Siraitia grosvenorii* genome, Monk fruit (Qingpiguo) v1 Genome | CuGenDBv2 (cucurbitgenomics.org). Although 30565 conserved protein regions can be found according to this genome, it still consisted of over 4000 contigs. This assembly had not reached the chromosome level. So, it was difficult to locate any trait gene on this genome. The applicant used third-generation long sequencing technology and the second-generation sequencing technology to sequence and assemble a male commercial variety, and finally assembled the *Siraitia grosvenorii* genome completely.

### c. Association analysis of GWAS in female and male traits

High-throughput second-generation sequencing was used to sequence the above 125 *Siraitia grosvenorii* germplasm resources, including 102 female plants and 17 male plants, and the SNP polymorphism of all germplasm was analyzed. An SNP database of 6.4 M loci was integrated. Using these SNP loci and the known sex traits, a genome-wide association analysis was conducted on this database.

Genome-wide association analysis (GWAS) refers to the method of using high-throughput sequencing technology to identify the existing sequence variations, i.e. SNPs, across the entire genome for genotyping, and then using bioinformatics methods to screen out SNP loci associated with complex traits or measurable phenotypes. GWAS is currently the most commonly used method for discovering functional gene marker.

In GWAS analysis, the GLM+3pc method was used to calculate the association analysis of SNPs. The mixed linear model algorithm (Yu et al., 2006) was used in GWAS analysis, which was implemented in the rMVP R software package v.1.0.6 (Yin et al., 2021). In this analysis, the applicant calculated the kinship matrix (calculated according to the method described in VanRaden, 2008) and the first three principal component variations (calculated as described above) of all varieties of Monk Fruit Corp. as covariates. The calculation of local linkage imbalance was performed using Plink v.1.9 software (Purcell et al., 2007).

GLM+3pc is a commonly used statistical model in genome-wide association studies (GWAS), that considers the influence of population structure on genetic variation. It uses three principal components to correct population stratification and false positives. It was mainly used to correct the influence of population structure on genetic variation, thereby correcting false positives. In GWAS, false positive refers to cases where no true association exists, but it is mistakenly identified as having an association due to the influence of the group structure. This situation may result in researchers wasting time and resources in searching for true associations. Compared with other methods of correcting population structure in GWAS, GLM+3pc is considered as a relatively simple and computationally efficient approach. It is also relatively easy to implement and interpret, which is why this method was chosen for calculation in this application.

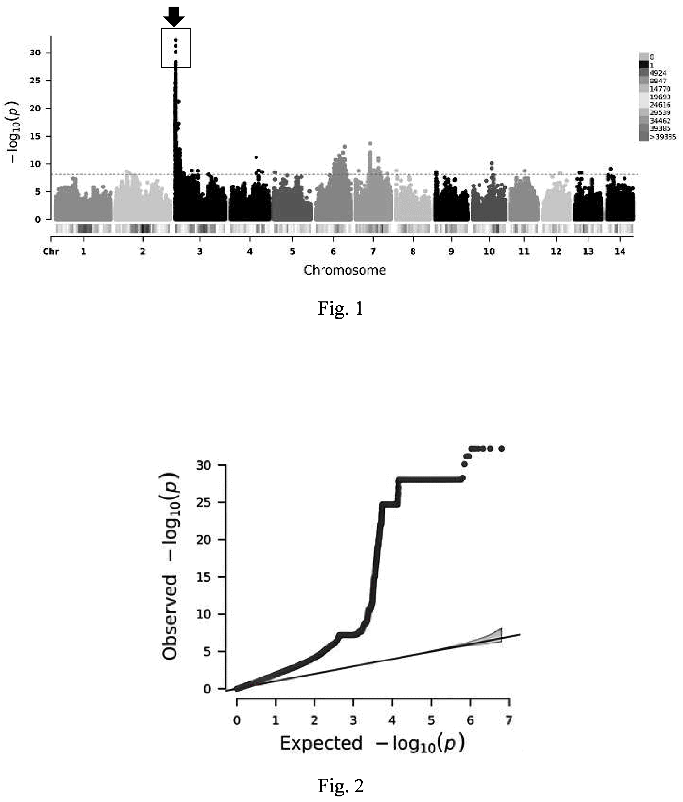

In GWAS analysis, the GWAS p-value (-log10(p)) of all SNPs within the region of chromosome 3 from 0 to 1mbp in consecutive segments showed that a gender-related trait is around the 0.49mb position. The Manhattan plot obtained from GWAS analysis is shown in Fig. 1, with the x-axis representing the base sequence from chromosomes 1-14. The y-axis -log₁₀(p) represents the statistical significance value of GWAS analysis, i.e. p-value.

In GWAS analysis, p-value is a statistical significance indicator that measures the association between genes or genetic variation and a trait. A smaller P-value indicates that the observed association results is unlikely to be due to random factors, increasing the confidence in rejecting the null hypothesis. However, P-values only provide association information. Although SNP loci with (-log10(p)) greater than or equal to 8 are theoretically considered to have significant associations with female and male, this application detected SNP loci with (-log10 (p)) greater than or equal to 25 as the most closely associated with female and male traits. Choosing any of these loci can distinguish between female and male plants.

The threshold was selected at (-log10(p)) greater than or equal to 25, that is, p-value greater than or equal to 25, for the sites. SNPs on chromosome 3 associated with gender traits were located in the peaks outlined in the figure, forming a series of female and male haploid SNP site sets. Any of these SNP loci can be used to distinguish between female and male plants, and these SNPs were referred to as golden SNPs.

At the same time, this application also conducted statistical analysis on the GWAS results. On chromosome 3, there were a total of 471 SNP loci with p-value greater than 25 and associated with gender traits. Any of these 471 SNP loci can be used to distinguish between female and male plants. Moreover, this application found that the larger the p-value, the higher the accuracy of its use for distinguishing between female and male plants. Through statistical analysis of the GWAS results, there were a total of 9 SNP loci on chromosome 3 with p-value greater than 30 and associated with gender traits, and these 9 SNP loci have higher accuracy for distinguishing between female and male plants.

By comparing the actual P-value of SNP loci with quantiles of probability value/expected values of the p-value of SNP loci, the comparison of two p-value probability distributions is achieved. As shown in Fig. 2, the Y-axis is the actual result of the p-value of SNP loci, i.e. the observed result; the X-axis is the probability value/expected value of uniform distribution, i.e. the expected result. The above indicators were all obtained after conversion to -log10.

Fig. 2 presents the distribution of P-values associated with female and male traits obtained from statistical tests in genome-wide association analysis (GWAS). The expected P-value was calculated under the null hypothesis and plotted on the x-axis, while the observed P-value was plotted on the y-axis. A straight line with a slope of one indicates that the observed p-value distribution conforms to the null hypothesis, while a curve indicates a distribution that deviates from the null hypothesis. The higher the degree of deviation, the higher the probability of association. As shown in Fig. 2, loci with -log10 (p) greater than 30 have the highest probability of association with female and male traits.

### d. Screening SNP Molecular Markers of Siraitia grosvenorii

One SNP locus with a high P-value was further screened from SNP loci with p-value greater than 30 and associated with gender traits, while meeting the requirements for designing allele-specific PCR with fluorescence endpoint method. The sequence of the SNP marker locus is shown in SEQ ID NO: 1:

ATTGTCGTCCTACCTATCTACAAAACAGCAGAGCCTTCAAAGTAGAAGCGGCTGCTT
TTACTGGATTATTAGAGAGAGAAGAAGTTGAGAAAATGAATGAACTGAGATTTGCTTGCA
TGCTSTCCATCCCCAATGGTTGATTTAATTCGTTTGGTAGGCAGAGATTGTAAAACATATAA
ACGAAATCTTTTGGGACATGATAAAAAATTAAATTTTCAGTTCCATGCTTTGGGTTGGGCC
CGTCATATTCTTATTCAACACACGTACGGGGCGCCT

In SEQ ID NO: 1, the nucleotide represented by S at position 122 was G or C, marked as [G/C], which was the SNP marker site. The polymorphism of the sequence in female and male plants was characterized by: when the alleles at this position in the diploid of *Siraitia grosvenorii* were G and C, the *Siraitia grosvenorii* was a male plant; when the alleles at this position in the diploid of *Siraitia grosvenorii* were C and C, the *Siraitia grosvenorii* was a female plant, i.e.,
G: C was male,
C: C was female.

### Example 2. Verification and Application of SNP Molecular Markers

the above markers were verified in 2019, 2020, and 2021 respectively. The methods used for verification were as follows:
1.Seedling cultivation, selecting tender sprouts for DNA extraction.
2. Extracting and purifying DNA using the most commonly used CTAB method for plant species.
3. Amplifying nucleotide fragments containing SNP loci and detecting amplification with specific primers.

The primer design for allelic PCR using the fluorescence endpoint method for the sequence of SEQ ID NO: 1 was as follows:
forward primer 1 (SEQ ID NO:2):
   5'-GAAGGTGACCAAGTTCATGCTTCAACCATTGGGGATGGAG-3' ;
forward Primer 2 (SEQ ID NO:3):
   5'-GAAGGTCGGAGTCAACGGATTTCAACCATTGGGGATGGAC-3' ;
reverse primer (SEQ ID NO:4):
   5'-GTAGAAGCGGCTGCTTTTACTGG-3'.

The above three primers were mixed in the following ratio: 12 µl of the forward primer 1, 12 µl of the forward primer 2, 30 µl of the reverse primer, and 46 µl of water to form a 100µl primer mixture.

The primer mixture (0.07 µl), DNA template (2.5 µl), and fluorescent PCR mixture (2.5 µl) were mixed and placed in a fluorescent PCR instrument, and the reaction was performed according to the following cycle parameters: (1) denaturation: 94°C, 15 minutes, 1 cycle; (2) specific cycle: 94°C, 20 seconds, 61°C-55°C, decreasing by 0.6°C, 10 cycles; (3) amplification cycle: 94°C, 20 seconds, 55°C, 60 seconds, 26 cycles; (4) Reading cycle: 30 °C, 60 seconds, 1 cycle. The nucleotide fragment comprising the SNP to be tested was amplified.

### 4. High throughput sequencing

After the PCR reaction, second-generation sequencing method was used to conduct high-throughput DNA detection on the nucleotide fragments of the above amplified SNPs to be tested of the 125 *Siraitia grosvenorii* varieties, so as to achieve early detection of female and male plants. The results were shown in Table 1 below.

In 2020, the same method as in 2019 was used for validation, with a total sample size of 194. The results are shown in Table 1 below.

In 2021, the same method as in 2019 was used for validation, with a total sample size of 383. The results are shown in Table 1 below.

**Table 1**

| **Genotype** | **Female** | **Male** | **Total** | **Prediction accuracy** |
|---|---|---|---|---|
| **2019** | **88** | **15** | **103** | 96.1% |
| C:C | 85 | 1 | 86 | |
| G:C | 3 | 14 | 17 | |
| **2020** | **81** | **113** | **194** | 94.8% |
| C:C | 76 | 5 | 81 | |
| G:C | 5 | 108 | 113 | |
| **2021** | **136** | **247** | **383** | 96.6% |
| C:C | 132 | 9 | 141 | |
| G:C | 4 | 238 | 242 | |
| **Total** | **305** | **375** | **680** | average value 95.9% |

Test results for the experimental seedlings from 2019 to 2021, as verified from Table 1, confirmed that the prediction accuracy of this marker to be over 94.8%.

*Siraitia grosvenorii* is a perennial vine, and it took 2-3 years to select and breed seedlings. The process of collection, cultivation, testing, and revalidation in this application took a lot of time and manpower. The development of this marker has expanded the scale of breeding from a selection of dozens of plants to thousands, increasing the scale of breeding selection by dozens of times and greatly improving the efficiency of breeding. These sequencing, planting, and final validation processes were carried out as accurately as possible by the applicant, resulting in a high prediction accuracy. Thus it indicates that the method of this application has high accuracy and efficiency.

This application has verified the SNP markers of this application in 2019, 2020, and 2021, providing stable, fast, and accurate predictions for three consecutive years. Such a lengthy verification process is very rare in this field, as it requires extensive experimental means and human and material resources. The SNP markers provided in this application have reached an accuracy of 96.1% in the validation process of 2019, which is the first time in this field to rapidly detect sex traits with such precision. However, with a rigorous and conscientious attitude, this application conducted another two years of verification, spending a lot of human and material resources. The facts have proven that the accuracy of the SNP markers provided in this application was very high, which is currently an unattainable achievement in this field.

Although the embodiments of the present application have been described in conjunction with the above, the present application is not limited to the specific embodiments and application field mentioned above. The specific embodiments mentioned above are only illustrative and instructive, and not restrictive. Under the inspiration of this specification and without departing from the scope of protection of the claims of the present application, ordinary skilled in the art can make many forms, all of which fall within the protection of this application.

## Claims

1. A SNP marker used for identifying female and male *Siraitia grosvenorii* plants, wherein, the SNP marker is a locus with a p-value greater than 25.

2. The SNP marker according to claim 1, wherein the nucleotide sequence of the SNP marker is as shown in SEQ ID NO: 1, located at position 122 of the nucleotide sequence, and the nucleotide represented by S in SEQ ID NO: 1 is G or C.

3. The SNP marker according to claim 1 or 2, wherein, when the alleles at the diploid S position of the *Siraitia grosvenorii* are G and C, the *Siraitia grosvenorii* is a male plant, and when the alleles at the diploid S position of the *Siraitia grosvenorii* are C and C, the *Siraitia grosvenorii* is a female plant.

4. Primers for detecting the SNP marker according to any one of claims 1-3, comprising, a forward primer 1:
5'-GAAGGTGACCAAGTTCATGCTTCAACCATTGGGGATGGAG-3' ;
a forward Primer 2:
5'-GAAGGTCGGAGTCAACGGATTTCAACCATTGGGGATGGAC-3' ; and
a reverse primer:
5'-GTAGAAGCGGCTGCTTTTACTGG-3'.

5. A detection reagent or kit for detecting a SNP marker according to any one of claims 1-3, comprising the primer according to claim 4.

6. Use of the SNP marker of any one of claims 1 to 3, the primers of claim 4, or the detection reagent or kit of claim 5 in identifying or screening female and male plants of *Siraitia grosvenorii.*

7. Use of the SNP marker of any one of claims 1 to 3, the primers of claim 4 or the detection reagent or kit of claim 5 in the marker assisted breeding of *Siraitia grosvenorii.*

8. Use of the SNP marker of any one of claims 1 to 3, the primers of claim 4 or the detection reagent or kit of claim 5 in the hybrid breeding of *Siraitia grosvenorii.*

9. A method for distinguishing between female and male plants of *Siraitia grosvenorii,* wherein the SNP marker was used for distinguishing between female and male plants of *Siraitia grosvenorii* in a test sample of *Siraitia grosvenorii* to predict whether the *Siraitia grosvenorii* is female or male plant, wherein the SNP marker is the SNP marker of any one of claims 1 to 3.

10. The method according to claim 9, comprising the following steps,
extracting the genomic DNA of *Siraitia grosvenorii* to be tested;
using the genomic DNA of the *Siraitia grosvenorii* to be tested as a template, perform PCR amplification with the primers of claim 4 to obtain the PCR amplification product;
detecting the PCR amplification product, if the alleles at the diploid S position of the amplification product sequence are G and C, the *Siraitia grosvenorii* is a male plant; when the alleles at the diploid S position of the *Siraitia grosvenorii* diploid are C and C, the *Siraitia grosvenorii* is a female plant.
